Europäisches Patentamt

European Patent Office

Office européen des brevets

(11) Veröffentlichungsnummer : **0 107 046**
**B1**

# (12) EUROPÄISCHE PATENTSCHRIFT

(45) Veröffentlichungstag der Patentschrift :
06.05.87

(51) Int. Cl.⁴ : **B 01 D 53/14**

(21) Anmeldenummer : 83109427.1

(22) Anmeldetag : 22.09.83

(54) **Verfahren zum Entfernen von CO2 und gegebenenfalls H2S aus Erdgasen.**

(30) Priorität : 02.10.82 DE 3236600

(43) Veröffentlichungstag der Anmeldung :
02.05.84 Patentblatt 84/18

(45) Bekanntmachung des Hinweises auf die Patenterteilung : 06.05.87 Patentblatt 87/19

(84) Benannte Vertragsstaaten :
AT BE DE FR GB IT NL SE

(56) Entgegenhaltungen :
DE-A- 2 551 717
GWF-GAS/ERDGAS, Band 123, Nr. 1, 1982 G. GROTE-
WOLD "Waschprozesse für die Reinigung und Konditionierung von sauren Erdgasen", Seiten 7-12
Chemie-Ingenieur-Technik, Band 53, Nr. 2, 1981-Sei-
ten 73-81

(73) Patentinhaber : BASF Aktiengesellschaft
Carl-Bosch-Strasse 38
D-6700 Ludwigshafen (DE)

(72) Erfinder : Wagner, Eckhart, Dr.
Jakobsgarten
D-6700 Ludwigshafen (DE)
Erfinder : Volkamer, Klaus, Dr.
Heidelberger Ring 21
D-6710 Frankenthal (DE)
Erfinder : Wagner, Ulrich, Dr.
Knospstrasse 7
D-6703 Limburgerhof (DE)

**0 107 046**

**Beschreibung**

Die vorliegende Erfindung betrifft ein Verfahren zum Entfernen von $CO_2$ und gegebenenfalls $H_2S$ aus Erdgasen mittels einer wäßrigen Absorptionsflüssigkeit.

Es ist bekannt, z. B. aus A.L. Kohl — F.C. Riesenfeld, Gas Purification, 3. Auflage, 1979, wäßrige Lösungen von Monoethanolamin oder Diethanolamin oder Mischungen von Cyclotetramethylensulfon und einer wäßrigen Lösung von Diisopropanolamin als Lösungsmittel zur Entfernung von $CO_2$ und gegebenenfalls $H_2S$ aus Erdgasen zu verwenden. Bei diesen Verfahren ist es erforderlich, das mit $CO_2$ und gegebenenfalls $H_2S$ beladene Lösungsmittel in einer Ausstreifkolonne durch Zufuhr von Dampf zu regenerieren, was mit erheblichem Energieaufwand verbunden ist. Bei der Entfernung von $CO_2$ und gegebenenfalls $H_2S$ aus höhere Kohlenwasserstoffe enthaltenden Erdgasen mittels einer Mischung von Cyclotetramethylensulfon und einer wäßrigen Lösung von Diisopropanolamin kommt als zusätzlicher Nachteil hinzu, daß die höheren Kohlenwasserstoffe in diesem Lösungsmittel eine relativ hohe Löslichkeit aufweisen, so daß das am Kopf der Ausstreifkolonne abgezogene Sauergas einen relativ hohen Gehalt an Kohlenwasserstoffen aufweist, der, falls das Sauergas $H_2S$ enthält, zu Schwierigkeiten in einer nachgeschalteten Claus-Anlage führen kann. Primäre oder sekundäre Alkanolamine, wie Monoethanolamin oder Diethanolamin, können außerdem nur als wäßrige Lösungen mit verhältnismäßig geringer Konzentration an diesen Alkanolaminen verwendet werden, da bei Verwendung höherer Konzentrationen schwere Korrosionsschäden an Anlageteilen auftreten können.

Weiter ist aus der DE-A-2 551 717 ein Verfahren zur Entfernung von $CO_2$ und/oder $H_2S$ aus Gasen unter Verwendung von Piperazin enthaltenden chemischen oder physikalischen Lösungsmitteln bekannt. Bei den chemischen Lösungsmitteln wird auch die Verwendung von Methyldiethanolamin (MDEA) beschrieben. Bei dem Verfahren wird das in einer einstufigen Wäsche aus der Absorptionsstufe erhaltene beladene Lösungsmittel zunächst durch Entspannung regeneriert und dann in einer zweiten Regenerierungsstufe in einer Ausstreifzone weiter regeneriert. Dieses Verfahren weist einen relativ hohen Dampfverbrauch auf.

Außerdem wird in Chemie-Ingenieur-Technik Band 53, Nr. 2, 1981, Seiten 73-81 ein Verfahren zum Entfernen von $CO_2$ aus Gasen beschrieben, bei dem in einer zweistufigen chemischen Wäsche das beladene Lösungsmittel zunächst in zwei Entspannungsstufen entspannt und anschließend in einem Ausstreifer weiter regeneriert wird.

Schließlich wird in EP-A-107 783 (Priorität : 02.10.1982 ; Anmeldetag : 22.09.1983 ; Veröffentlichungstag : 09.05.1984) ein Verfahren zum Entfernen von $CO_2$ und/oder $H_2S$ aus Gasen mittels einer Methyldiethanolamin enthaltenden wäßrigen Absorptionsflüssigkeit in einer einstufigen Wäsche beschrieben, bei dem die Regenerierung der beladenen Absorptionsflüssigkeit ausschließlich in einer oder mehreren Entspannungsstufen durchgeführt wird, wobei zum Ausgleich der Wasserverluste durch in den am Kopf der Absorptionsstufe und der Entspannungsstufe bzw. Entspannungsstufen abgezogenen Gasströmen enthaltenes Wasser am Sumpf der letzten Entspannungsstufe, bzw., falls nur eine Entspannungsstufe verwendet wird, am Sumpf dieser einen Entspannungsstufe eine dem Wasserverlust entsprechende Menge Wasserdampf zugeführt wird.

Es bestand daher Bedarf nach einem Verfahren zum Entfernen von $CO_2$ und gegebenenfalls $H_2S$ aus Erdgasen, bei dem die Nachteile der bekannten Verfahren vermieden werden können.

Es wurde nun ein vorteilhaftes Verfahren gefunden zum Entfernen von $CO_2$ und gegebenenfalls $H_2S$ aus $CO_2$ und gegebenenfalls $H_2S$ enthaltenden Erdgasen, bei dem man die $CO_2$ und gegebenenfalls $H_2S$ enthaltenden Erdgase in einer Absorptionsstufe bei Temperaturen von 40 bis 100 °C mit einer 20 bis 70 Gew.-% Methyldiethanolamin enthaltenden wäßrigen Absorptionsflüssigkeit behandelt, am Kopf der Absorptionsstufe die behandelten Erdgase abzieht, am Sumpf der Absorptionsstufe die mit $CO_2$ und gegebenenfalls $H_2S$ beladene wäßrige Absorptionsflüssigkeit abzieht und anschließend regeneriert und die regenerierte Absorptionsflüssigkeit in die Absorptionsstufe zurückführt, welches dadurch gekennzeichnet ist, daß man die Regenerierung der beladenen Absorptionsflüssigkeit ausschließlich in einer oder mehreren Entspannungsstufen durchführt.

Nach dem neuen Verfahren wird das mit $CO_2$ und gegebenenfalls $H_2S$ beladene Lösungsmittel ohne Verwendung einer Ausstreifkolonne allein durch Entspannung in einer oder mehreren Entspannungsstufen regeneriert. Durch den Wegfall der Ausstreifkolonne kann der apparative Aufwand bei gleichzeitigem Wegfall der Zuführung von Dampf für den Ausstreifvorgang erheblich verringert werden, so daß eine erhebliche Reduktion sowohl der Investitionskosten als auch der Energieverbrauchskosten erreicht wird. Weiter können bei dem neuen Verfahren relativ hohe Methyldiethanolaminkonzentrationen in der Absorptionsflüssigkeit verwendet werden, ohne daß es zu Korrosionsschäden in der Gaswaschanlage kommt.

Das erfindungsgemäße Verfahren wird vorteilhaft zum Entfernen von $CO_2$ und gegebenenfalls $H_2S$ aus Erdgasen verwendet, die neben Methan noch höhere Kohlenwasserstoffe enthalten. Bei diesen höheren Kohlenwasserstoffen handelt es sich im allgemeinen um $C_2$- bis $C_{30}$-Kohlenwasserstoffe, vorzugsweise $C_2$- bis $C_{20}$-Kohlenwasserstoffe, insbesondere $C_2$- bis $C_{12}$-Kohlenwasserstoffe, die in der Regel aliphatisch sind, z. B. Ethan, Propan, Isobutan, n-Butan, Isopentan, n-Pentan, die Hexane, Heptane, Octane, Nonane, Decane und die höheren Homologen. Die höheren Kohlenwasserstoffe können neben

2

den aliphatischen Kohlenwasserstoffen auch noch aromatische Kohlenwasserstoffe wie Benzol enthalten. Im allgemeinen beträgt der Gehalt der Erdgase an den höheren Kohlenwasserstoffen 0,1 bis 40 Mol%, vorzugsweise 0,5 bis 30 Mol%, insbesondere 1 bis 20 Mol%.

Die Erdgase weisen im allgemeinen einen $CO_2$-Gehalt von 1 bis 90 Mol%, vorzugsweise 2 bis 90 Mol%, insbesondere 5 bis 60 Mol% auf. Neben dem $CO_2$ können die Erdgase als weiteres Sauergas $H_2S$ enthalten, z. B. in Mengen von wenigen Mol.-ppm, beispielsweise 1 Mol.-ppm, bis 50 Mol.%.

Als Lösungsmittel wird für das erfindungsgemäße Verfahren eine 20 bis 70 Gew.-%, vorzugsweise 30 bis 65 Gew.-%, insbesondere 40 bis 60 Gew.-% Methyldiethanolamin enthaltende wäßrige Absorptionsflüssigkeit verwendet. Zweckmäßig wird eine wäßrige Methyldiethanolaminlösung eingesetzt, z. B. eine wäßrige Lösung von Methyldiethanolamin technischer Reinheit. In einer vorteilhaften Ausführungsform des Verfahrens verwendet man eine wäßrige Methyldiethanolaminlösung, die zusätzlich 0,1 bis 1 Mol/l, vorzugsweise 0,2 bis 0,8 Mol/l, insbesondere 0,25 bis 0,6 Mol/l eines sekundären Amins oder Alkanolamins, vorzugsweise Methylmonoethanolamin, ganz besonders vorteilhaft Piperazin, enthält.

Das erfindungsgemäße Verfahren wird in der Weise durchgeführt, daß das $CO_2$ und gegebenenfalls $H_2S$ enthaltende Erdgas zunächst in einer Absorptionsstufe mit der Methyldiethanolamin enthaltenden Absorptionsflüssigkeit behandelt wird. Dabei werden in der Absorptionsstufe Temperaturen von 40 bis 100 °C, vorzugsweise 50 bis 90 °C, insbesondere 60 bis 80 °C für die Absorptionsflüssigkeit aufrechterhalten. Im allgemeinen werden in der Absorptionsstufe Drucke von 10 bis 110 bar, vorzugsweise 20 bis 100 bar, insbesondere 30 bis 90 bar angewendet. Als Absorptionsstufe wird zweckmäßig eine Absorptionskolonne verwendet, im allgemeinen eine Füllkörperkolonne oder eine mit Böden ausgestattete Kolonne. Zweckmäßig wird das zu behandelnde Erdgas am Sumpf und die Absorptionsflüssigkeit am Kopf der Absorptionskolonne zugeführt, wobei die sauren Gase $CO_2$ und, falls im Erdgas enthalten, $H_2S$ im Gegenstrom ausgewaschen werden. Während gegebenenfalls vorhandenes $H_2S$ zweckmäßig weitgehend, im allgemeinen auf $H_2S$-Gehalte im behandelten Erdgas von höchstens 120 Mol.-ppm, vorzugsweise höchstens 10 Mol.-ppm, insbesondere höchstens 3 Mol.-ppm ausgewaschen wird, kann es vorteilhaft sein, das $CO_2$ im Erdgas soweit auszuwaschen, daß die $CO_2$-Gehalte im behandelten Erdgas höchstens etwa 0,5 bis 6, vorzugsweise 0,5 bis 5, insbesondere 1 bis 4 Mol.% betragen. Das behandelte Erdgas wird zweckmäßig am Kopf der Absorptionsstufe, zweckmäßig an einem Punkt, der oberhalb der Zuführung der Absorptionsflüssigkeit liegt, abgezogen. Die mit den Sauergasen $CO_2$ und gegebenenfalls $H_2S$ beladene Absorptionsflüssigkeit wird zweckmäßig am Sumpf der Absorptionszone abgezogen.

Anschließend wird die beladene Absorptionsflüssigkeit in ein oder mehreren Entspannungsstufen regeneriert. Dabei wird in der letzten Entspannungsstufe bzw., falls nur eine Entspannungsstufe verwendet wird, in dieser einzelnen Entspannungsstufe zweckmäßig auf einen Druck von etwa 1 bis 3 bar, vorzugsweise 1 bis 1,8 bar, insbesondere 1 bis 1,5 bar entspannt. Es kann auch zweckmäßig sein, die letzte Entspannungsstufe bzw. die einzelne Entspannungsstufe unter vermindertem Druck gegenüber Atmosphärendruck, z. B. bei Drucken von 0,5 bis etwa 1 bar, vorzugsweise von 0,8 bis etwa 1 bar, zu betreiben. Es kann vorteilhaft sein, mindestens zwei Entspannungsstufen, z. B. 2 bis 5, vorzugsweise 2 oder 3, insbesondere 2 Entspannungsstufen, für die Regenerierung der beladenen Absorptionsflüssigkeit zu verwenden. Vorzugsweise wird dabei das beladene Absorptionsmittel in der ersten Entspannungsstufe nach der Absorptionsstufe auf einen Druck entspannt, der mindestens 5 bar beträgt. Es kann dabei vorteilhaft sein, daß der Druck, auf den in der ersten Entspannungsstufe nach der Absorptionsstufe entspannt wird, zusätzlich mindestens der Summe der Partialdrucke an $CO_2$ und gegebenenfalls $H_2S$ in dem der Absorptionsstufe zugeführten, $CO_2$ und gegebenenfalls $H_2S$ enthaltenden Erdgas ist. Bei dieser Arbeitsweise können die Verdampfung von Wasser und die damit verbundenen Energieverluste sowie die Verluste an Kohlenwasserstoffen besonders niedrig gehalten werden. Für die Entspannung werden zweckmäßig Entspannungsbehälter, die z. B. auch als Kolonnen gestaltet sein können, verwendet. Diese Entspannungsbehälter können frei von besonderen Einbauten sein. Es können jedoch auch mit Füllkörpern oder Austauschböden ausgestattete Kolonnen verwendet werden.

In der Regel wird zum Ausgleich von verfahrensbedingten Wärmeverlusten, die beispielsweise durch die Entspannungsverdampfung bewirkt werden, dem Verfahren Wärme zugeführt. Dies erfolgt zweckmäßig in einer vor die letzte Entspannungsstufe bzw., falls nur eine Entspannungsstufe verwendet wird, vor diese einzelne Entspannungsstufe geschalteten Wärmeaustauschzone, in der die beladene Absorptionsflüssigkeit vor der Entspannung in der letzten bzw. einzelnen Entspannungsstufe erwärmt wird. In der Regel wird die Absorptionsflüssigkeit in der Austauschzone um maximal 20 °C erwärmt, wobei die Absorptionsflüssigkeit im allgemeinen auf eine Temperatur von höchstens 90 °C, in der Regel von höchstens 85 °C erwärmt wird. Für die Wärmeaustauschzone werden im allgemeinen Wärmetauscher, z. B. ein Röhrenwärmetauscher, verwendet.

Die Sauergase $CO_2$ und gegebenenfalls $H_2S$ werden zweckmäßig am Kopf der letzten Entspannungsstufe abgezogen. Falls das abgezogene Sauergas $H_2S$ enthält, wird es zweckmäßig durch Oxidation des $H_2S$, z. B. in einer Claus-Anlage, aufgearbeitet. Die am Sumpf der letzten Entspannungsstufe abgezogene regenerierte Absorptionsflüssigkeit wird in die Absorptionszone zurückgeführt.

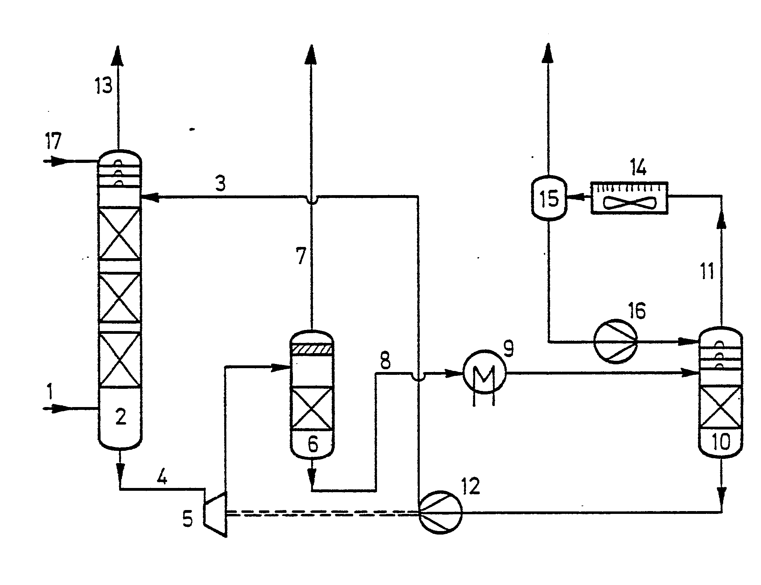

Nachfolgend werden weitere Einzelheiten der Erfindung anhand eines Ausführungsbeispiels, dessen Verfahrensablauf in der Figur schematisch dargestellt ist, erläutert.

Ein $CO_2$ und gegebenenfalls $H_2S$ und höhere Kohlenwasserstoffe, z. B. aliphatische $C_2$- bis $C_{10}$-Kohlenwasserstoffe, enthaltendes Erdgas wird über Leitung 1 unter Druck in den Sumpf der Absorptionskolonne 2 gegeben. Gleichzeitig wird über Leitung 3 als Absorptionsflüssigkeit 20 bis 70 gew.-%ige wäßrige Methyldiethanolaminlösung auf den Kopf der Absorptionskolonne gegeben. Die Absorptionsflüssigkeit, die im Gegenstrom zum Erdgas geführt wird, belädt sich mit den sauren Gasen $CO_2$ und, falls im zu waschenden Erdgas vorhanden, $H_2S$, und die beladene Absorptionsflüssigkeit wird über Leitung 4 am Sumpf der Absorptionskolonne abgezogen. Das gewaschene Erdgas wird über Leitung 13 am Kopf der Absorptionskolonne abgezogen. Der beladene Absorptionsflüssigkeitsstrom 4 wird anschließend in einen. Entspannungsverdampfungsbehälter 6 auf einen Druck von mindestens 5 bar entspannt, beispielsweise über ein Ventil oder vorzugsweise über eine Entspannungsturbine 5. Hierbei wird ein Kohlenwasserstoffe und $CO_2$ enthaltendes Zwischenentspannungsgas aus der Absorptionsflüssigkeit freigesetzt, das über Leitung 7 am Kopf des Entspannungsbehälters 6 abgezogen wird. Am Boden des Entspannungsbehälters 6 wird die teilentspannte Absorptionsflüssigkeit über Leitung 8 abgezogen, in Wärmetauscher 9 erwärmt, z. B. um 1 bis 15 °C, und die erwärmte Absorptionsflüssigkeit wird in einen zweiten Entspannungsbehälter 10 entspannt, z. B. auf einen Druck von 1 bis 2 bar. Hierbei wird ein $CO_2$-reiches Entspannungsgas, z. B. mit einer $CO_2$-Konzentration von 98 Mol.%, freigesetzt, das über Leitung 11 am Kopf des Entspannungsbehälters 10 abgezogen wird. Die am Sumpf des Entspannungsbehälters 10 abgezogene regenerierte Absorptionsflüssigkeit wird mit Hilfe einer Umlaufpumpe 12 zum Kopf der Absorptionskolonne 2 zurückgeführt. Zweckmäßig wird das über Leitung 11 abgezogene Gas im Wärmetauscher 14 zur Kondensation von durch die Entspannung freigesetztem Wasserdampf gekühlt. Das sich dabei im Trenngefäß 15 abscheidende Wasser wird über Pumpe 16 auf den Kopf des Entspannungsbehälters 10 zurückgeführt. Im allgemeinen wird am Kopf der Absorptionskolonne 2 über Leitung 17 Wasser zugeführt, um durch eine Wasserwäsche den Verlust an Methyldiethanolamin gering zu halten.

Das folgende Beispiel veranschaulicht die Erfindung.

## Beispiel

In einer Absorptionskolonne werden 3,15 kmol/h eines $CO_2$-haltigen Erdgases mit einer 50 gew.-%igen wäßrigen Methyldiethanolamin-Lösung als Absorptionsflüssigkeit bei einem Druck von 75 bar gewaschen. Das zu reinigende Gas hat folgende Zusammensetzung :

| | |
|---|---|
| $CO_2$ | 10,0 Mol.% |
| $CH_4$ | 75,0 Mol.% |
| höhere Kohlenwasserstoffe ($C_2$- bis $C_{12}$-Kohlenwasserstoffe) | 15,0 Mol.% |

Die Temperatur des Absorptionsmittels im Zulauf zur Absorptionskolonne beträgt 70 °C. Der $CO_2$-Gehalt im gewaschenen Gas beträgt 2,0 Mol.%. Das die Absorptionskolonne verlassende beladene Waschmittel wird in einem ersten Entspannungsbehälter auf einen Druck von 20 bar entspannt. Hierbei werden 0,04 kmol/ltr eines kohlenwasserstoffreichen Zwischenentspannungsgases mit einer $CO_2$-Konzentration von 34,3 Mol.% aus der Lösung freigesetzt und am Kopf des ersten Entspannungsbehälters abgezogen. Die teilentspannte Absorptionsflüssigkeit wird anschließend in einem Wärmetauscher um ca. 5 °C erwärmt. Die erwärmte Absorptionsflüssigkeit wird in einem zweiten Entspannungsbehälter auf 1,3 bar entspannt. Hierbei werden 0,241 kmol/h eines $CO_2$-reichen Entspannungsgases mit einer $CO_2$-Konzentration von 97,55 Mol.%, einer Methankonzentration von 1,68 Mol.% und einer Konzentration an höheren Kohlenwasserstoffen von 0,77 Mol.% frei, die am Kopf des zweiten Entspannungsbehälters abgezogen werden. Die am Sumpf des Entspannungsbehälters abgezogene Absorptionsflüssigkeit wird mit Hilfe einer Umlaufpumpe zum Kopf der Absorptionskolonne zurückgepumpt.

In einem Vergleichsbeispiel wird der oben beschriebene Versuch unter den gleichen Bedingungen mit einer 30 gew.-%igen wäßrigen Monoethanolaminlösung wiederholt. Bei Verwendung der gleichen Wärmeleistung zur Aufheizung des Lösungsmittels, wie sie für die wäßrige Methyldiethanolaminlösung benutzt wird, läßt sich die $CO_2$-Konzentration im gewaschenen Gas auf lediglich 7 Mol.% verringern.

## Patentansprüche

1. Verfahren zum Entfernen von $CO_2$ und gegebenenfalls $H_2S$ aus $CO_2$ und gegebenenfalls $H_2S$ enthaltenden Erdgasen, bei dem man die $CO_2$ und gegebenenfalls $H_2S$ enthaltenden Erdgase in einer Absorptionsstufe bei Temperaturen von 40 bis 100 °C mit einer 20 bis 70 Gew.-% Methyldiethanolamin enthaltenden wäßrigen Absorptionsflüssigkeit behandelt, am Kopf der Absorptionsstufe die behandelten Erdgase abzieht, am Sumpf der Absorptionsstufe die mit $CO_2$ und gegebenenfalls $H_2S$ beladene wäßrige Absorptionsflüssigkeit abzieht und anschließend regeneriert und die regenerierte Absorptionsflüssigkeit in die Absorptionsstufe zurückführt, dadurch gekennzeichnet, daß man die Regenerierung der beladenen Absorptionsflüssigkeit ausschließlich in einer oder mehreren Entspannungsstufen durchführt.

2. Verfahren gemäß Anspruch 1, dadurch gekennzeichnet, daß es zum Entfernen von $CO_2$ und

gegebenenfalls H₂S aus neben Methan noch höhere Kohlenwasserstoffe enthaltenden Erdgasen verwendet wird.

3. Verfahren gemäß Anspruch 1 und 2, dadurch gekennzeichnet, daß mindestens zwei Entspannungsstufen für die Regenerierung des beladenen Absorptionsmittel verwendet werden und daß das beladene Absorptionsmittel in der ersten Entspannungsstufe nach der Absorptionsstufe auf einen Druck entspannt wird, der mindestens 5 bar beträgt.

### Claims

1. A process for removing $CO_2$ and, if present, $H_2S$ from a natural gas which contains $CO_2$ and may contain $H_2S$, in which the said natural gas is treated, in an absorption stage at from 40 to 100 °C, with an aqueous absorption liquid containing from 20 to 70 % by weight of methyldiethanolamine, the treated natural gas is taken off at the top of the absorption stage, the aqueous absorption liquid laden with $CO_2$ and any $H_2S$ is taken off at the bottom of the absorption stage and then regenerated, and the regenerated absorption liquid is recycled to the absorption stage, characterized in that the regeneration of the laden absorption liquid is effected exclusively in one or more flash stages.

2. A process as claimed in claim 1, characterized in that $CO_2$ and any $H_2S$ are removed from a natural gas containing higher hydrocarbons in addition to methane.

3. A process as claimed in claims 1 and 2, characterized in that at least two flash stages are used for regenerating the laden absorption agent, and the latter is let down to a pressure of at least 5 bar in the first flash stage after the absorption stage.

### Revendications

1. Procédé d'extraction du $CO_2$ et, le cas échéant, du $H_2S$ de gaz naturels contenant du $CO_2$ et éventuellement du $H_2S$, dans lequel les gaz naturels renfermant du $CO_2$ et du $H_2S$ éventuel sont traités dans un stade d'absorption, à des températures comprises entre 40 et 100 °C, avec une solution d'absorption aqueuse, contenant entre 20 et 70 % en poids de méthyl-diéthanol-amine, les gaz naturels traités étant soutirés au sommet de la zone d'absorption, tandis que la solution d'absorption aqueuse chargée du $CO_2$ et du $H_2S$ éventuel est soutirée à la base de la zone d'absorption et régénérée et la solution d'absorption régénérée est recyclée dans le stade d'absorption, caractérisé en ce que la régénération de la solution d'absorption chargée est exclusivement réalisée dans un ou plusieurs stades de détente.

2. Procédé suivant la revendication 1, caractérisé en ce qu'il est mis en œuvre pour l'extraction du $CO_2$ et éventuellement du $H_2S$ de gaz naturels contenant, à côté du méthane, des hydrocarbures supérieurs.

3. Procédé suivant la revendication 1 ou la revendication 2, caractérisé en ce que la régénération du produit d'absorption chargé est réalisée dans au moins deux stades de détente, le produit d'absorption chargé étant détendu dans le premier stade de détente en aval du stade d'absorption à une pression au moins égale à 5 bars.